# EUROPEAN PATENT APPLICATION

(11) **EP 3 400 867 A1**
(43) Date of publication of application: **14.11.2018**
(21) Application number: 17170217.8
(22) Date of filing: 09.05.2017
(51) Int. Cl.: A61B 5/0205, A61B 5/0215, A61M 39/10

(54) **SYSTEM FOR PERFORMING IN VIVO PRESSURE MEASUREMENTS AND COUPLING MODULE**

(71) Applicant: Holding Novio Pelvic B.V., 7602 CR Almelo (NL)
(72) Inventor: Dijkman, Gerrat, 7523 DX Enschede (NL)
(74) Representative: Mink-Lindenburg, Charlotte Hildegard

(57) **Abstract**

The invention relates to a system for performing in vivo pressure measurements, comprising one or more pressure sensors (14), a connector module (20) provided with one or more electrical coupling elements for the one or more pressure sensors and one or more coupling means (10) for a catheter. The one or more coupling means for a catheter are each provided with at least three ports and a tap for coupling two of the three ports as desired. According to the invention each of the one or more coupling means is embodied as a coupling module which is releasably connectable to the connector module, and the pressure sensors (14) are arranged on the coupling modules (10).

The invention also relates to a coupling module (10) for a catheter as described as component of the system (1) according to the invention.

## Description

The present invention relates to a system for performing in vivo pressure measurements, comprising one or more pressure sensors, a connector module provided with one or more electrical coupling elements for the one or more pressure sensors and one or more coupling means for a catheter, wherein the one or more coupling means for a catheter are each provided with at least three ports and a tap for coupling two of the three ports as desired.

Such a system is known from NL 1021054 of the same inventor. Described in NL 1021054 is a universal measuring device for medical application with a cassette which corresponds to the above stated system according to the preamble. The cassette is a disposable article intended for once-only use or use by one patient at most. The known measuring device hereby satisfies the highest requirements in respect of hygiene.

The invention has for its object to provide an improved system of the type stated in the preamble.

The system according to the present invention is distinguished from the known system in that each of the one or more coupling means is embodied as a coupling module which is releasably connectable to the connector module, and that the pressure sensors are arranged on the coupling modules.

In the system according to the invention each coupling module forms an separately disposable article. The coupling module comprises considerably fewer components than the known cassette, so that the amount of disposable material is considerably reduced. The number of coupling modules required for each patient can moreover be precisely adapted to the situation. In the system according to invention only the actually used and contaminated components are therefore thrown away after use. The environmental impact is hereby minimal. Since each coupling module in practice comprises only one pressure sensor, only actually used pressure sensors are discarded, this resulting in a cost-saving.

It is noted that US 2009/0069714 describes a system for performing in vivo pressure measurements wherein a pressure sensor housing is configured on the one hand for connection of a urine catheter and on the other is releasably connectable to a connector module by means of a plug. The coupling module of the system according to the invention is distinguished from the known pressure sensor housing in that it is provided with at least three ports and a tap for coupling two of the three ports as desired. The invention hereby provides a universally applicable measuring system for performing in vivo pressure measurements.

In a first preferred embodiment each coupling module is provided with a coupling arm which extends in line with one of the ports and is configured for locking to the connector module, and the pressure sensor is arranged on the coupling arm and configured for coupling to one of the electrical coupling elements on the connector module. In the first preferred embodiment the releasable connection takes a reliable and foolproof form as a result of a robust mechanical locking and a direct electrical connection of the pressure sensor. The coupling arm has a search function and guarantees a correct electrical connection between the coupling module and the connector module. By means of the locking the coupling arm guarantees a reliable mechanical connection between the coupling module and the connector module. Because of its position in line with one of the ports, the coupling arm moreover imparts support and robustness to means for fluid supply, such as hoses and valves, to be connected to the respective port.

In a further elaboration of the first preferred embodiment the pressure sensor is provided with coupling pins for insertion in the electrical coupling element. Because of the coupling pins soldering of electrical connections to for instance plugs is unnecessary.

In a further elaboration of the first preferred embodiment the connector module is provided with one or more openings for receiving the coupling arm, and the electrical coupling elements are arranged in the openings. A compact and highly reliable connection is hereby realized between the coupling module and the connector module.

In a subsequent preferred embodiment each coupling module comprises a snap-lock for releasable connection to the connector module. A snap-lock forms a reliable releasable connection which can be locked and unlocked easily, quickly and in error-free manner.

In a compact preferred embodiment the snap-lock is arranged close to the pressure sensor.

In a practical preferred embodiment the coupling module is embodied as two-way tap.

In a further user-friendly preferred embodiment one of the ports of one or more of the coupling modules is provided with a non-return valve.

In an optimal preferred embodiment the system further comprises a communication module for processing and/or transmitting measurement data from the one or more pressure sensors, which communication module is releasably connectable to the connector module. The communication module is preferably provided with a display.

The invention further relates to a coupling module for a catheter, which coupling module is embodied as two-way tap and is provided with at least three ports and a tap for coupling two of the three ports as desired, wherein the coupling module is provided with a coupling arm extending in line with one of the ports, and wherein a pressure sensor is arranged in the coupling arm.

In a first preferred embodiment of the coupling module the coupling arm is configured for releasable locking to a connector module so as to form a system for performing in vivo pressure measurements, wherein the connector module is provided with at least one electrical coupling element for the pressure sensor.

The invention will be elucidated on the basis of the preferred embodiments with reference to the drawings, in which:
Figure 1 is a schematic view of a first preferred embodiment of a system according to the invention;
Figure 2 shows a part of the preferred embodiment of figure 1 in exploded view;
Figure 3A shows another part of the preferred embodiment of figure 1 in exploded view;
Figure 3B shows a second preferred embodiment as alternative to figure 3A; and
Figure 3C shows a third preferred embodiment as alternative to figure 3A.

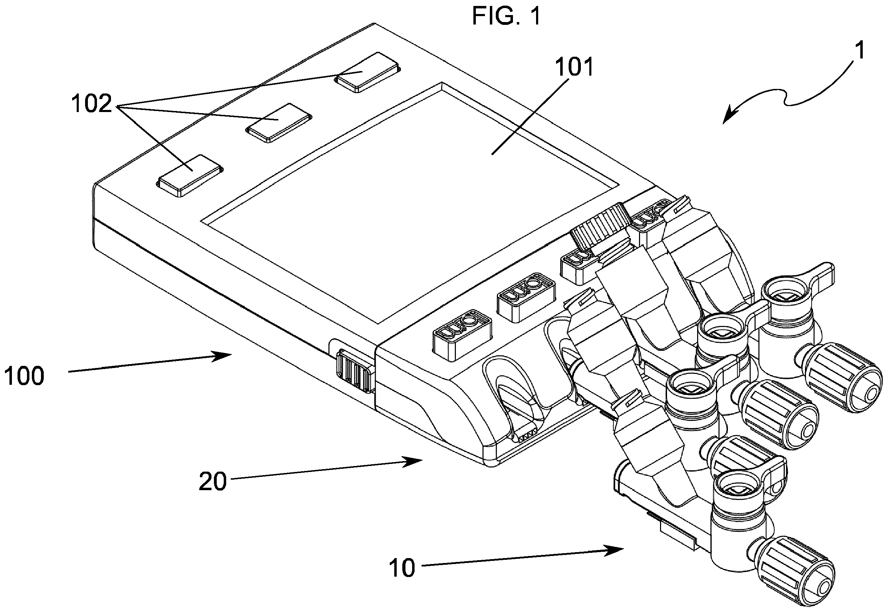

Figure 1 is a schematic view of a first preferred embodiment of a system 1 according to the invention. System 1 is a system for performing an in vivo pressure measurement. In the shown preferred embodiments system 1 is intended for the purpose of performing urodynamic measurements in vivo. System 1 comprises a communication module 100, a connector module 20 and at least one coupling module for a catheter in the form of a coupling tap 10.

Coupling taps 10 are each releasably connectable to connector module 20. The connection preferably takes place using a snap-lock. In the shown preferred embodiments connector module 20 and communication module 100 are also releasably connectable. This connection preferably takes place by means of at least one electrical connector 23 in combination with a mechanical connection of the respective housing parts. It is noted that many types of suitable connector are commercially available for releasable connection of connector module 20 and communication module 100.

During use coupling modules 10 come into contact with bodily fluids of the patient and are intended as a disposable article or for use with one patient. Connector module 20 and communication module 100 are intended for reuse.

It is noted that in the context of the invention it is not essential for connector module 20 to be releasably connected to communication module 100. If a fixed connection is opted for, there will be a connector part 20 and a communication part 100. It will however be assumed in the remainder of the figure description that the connection is releasable.

Communication module or communication part 100 is provided with a display 101 and control buttons 102 for communication with the user.

Communication module 100 is further provided with wireless communication means for transmitting measurement data to peripherals, for instance a computer. The communication means comprise at least one transmitter. All peripherals with a suitable receiver can receive and/or process the data sent by the communication module. Examples of such peripherals are computers, such as personal computers, including laptops, but also computers of an even more manageable size. The communication means can if desired also comprise a receiver. Suitable wireless communication means are per se known and available commercially. An example of suitable wireless communication means is the Blue Tooth® system or WiFi. Use is preferably made of a protocol for monitoring the completeness and/or reliability of the received data. Such protocols are per se known and available commercially in the form of software and/or hardware.

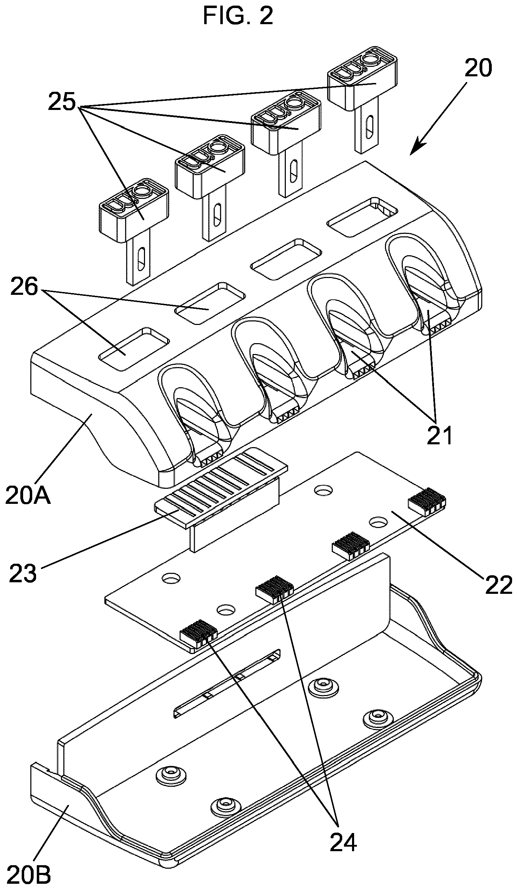

Figure 2 shows connector module or connector part 20 with exploded parts. Connector module 20 comprises two housing parts: an upper housing part 20A and a lower housing part 20B which together form a liquid-tight housing. Connector module 20 is provided with openings 21 for receiving coupling modules 10. Arranged in openings 21 are locking means which can be operated by means of release knobs 25. Release knobs 25 are placed through openings 26 of connector module 20.

Accommodated in the housing of connector module 20 is a printed circuit board (PCB) 22 on which electrical coupling elements 24 are arranged. The electrical coupling elements 24 are intended for measuring means 14 in coupling modules 10.

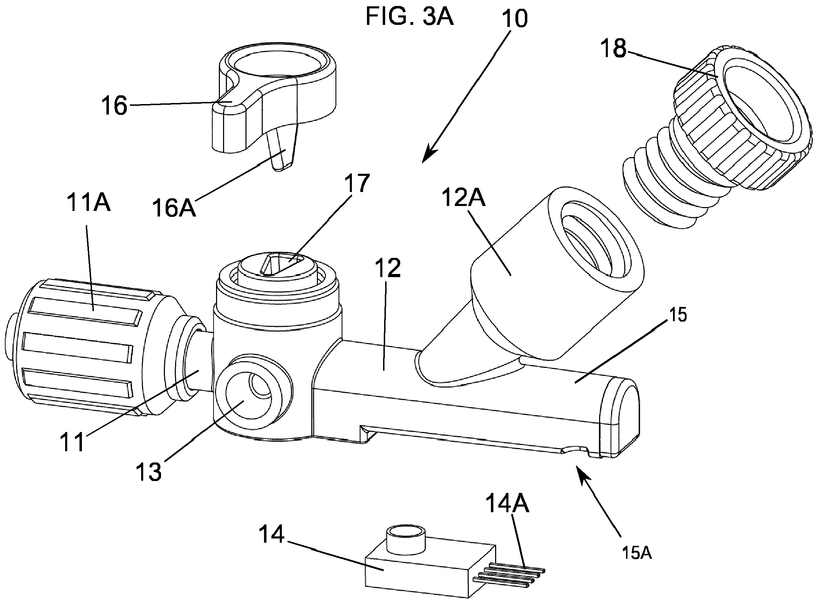

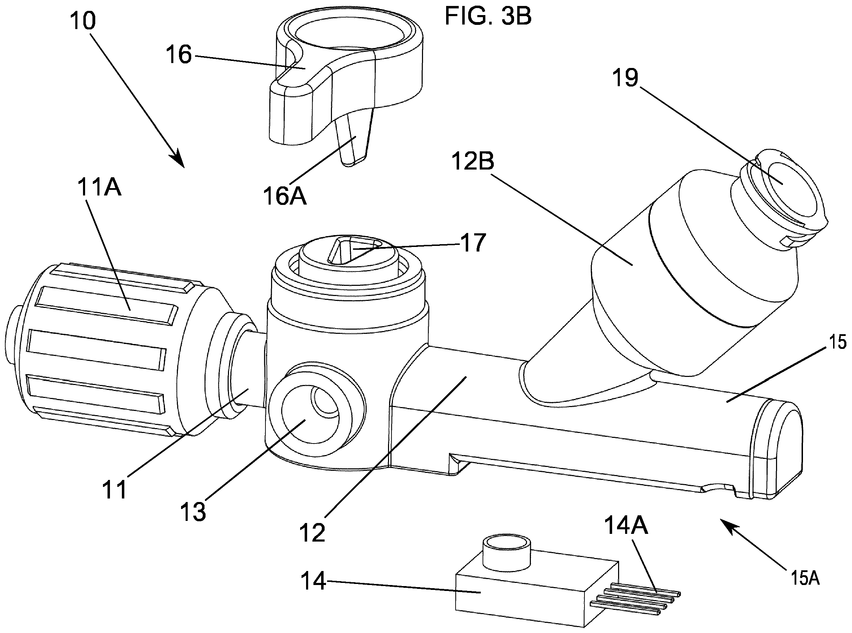

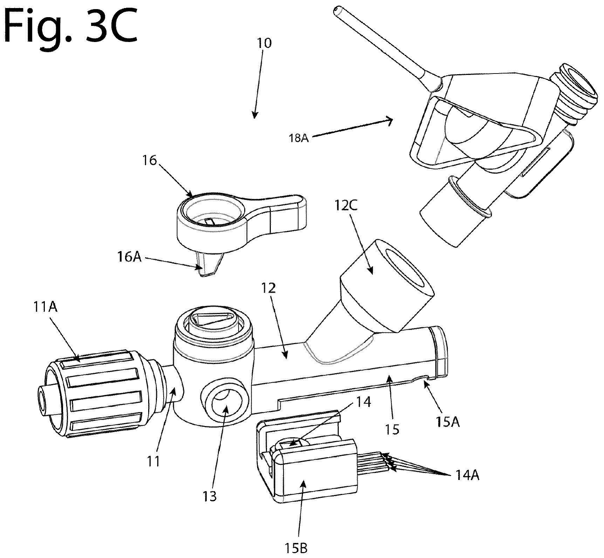

Figure 3A shows a first embodiment of a coupling module 10 intended for connection of a catheter filled with liquid. Figure 3B shows a second preferred embodiment of a coupling tap according to the invention intended for connection of a catheter filled with gas. Figure 3C shows a third preferred embodiment of a coupling tap according to the invention intended for connection of a flow reduction valve. The same components in figures 3A, 3B and 3C are provided with the same reference numerals. The difference between the three embodiments lies in the second port 12, and will be further elucidated below. The catheters to be connected are not shown.

Coupling module 10 is embodied as two-way tap and is therefore also referred to as coupling tap 10. Coupling tap 10 has a first port 11 for connection of a catheter. First port 11 is preferably provided with a Luer-type fitting 11A as known in the relevant field. The first port is connected as desired to a second port 12 in which measuring means 14 are accommodated, or a third port 13 for resetting purposes.

Present on coupling tap 10 is an operating handle or tap 16 for operation by the user. Tap 16 is releasably mountable and is provided for this purpose with a protrusion 16A intended to be received in a recess 17 on coupling tap 10.

In the first preferred embodiment second port 12 is embodied for liquid measurements, preferably using water. Liquid connection 12A, 18 is preferably provided with a non-return valve.

In the second preferred embodiment second port 12 is configured for gas measurements, preferably using air, and is provided with a gas connection 12B and 19.

In the third preferred embodiment second port 12 is embodied for liquid measurements, preferably using water. Liquid connection 12C is configured for connection of a flow reduction valve 18A.

Third port 13 provides the option of resetting to atmospheric pressure.

Each coupling module 10 is provided with a coupling arm or coupling bridge 15 configured for locking to connector module 20. In the shown preferred embodiment coupling arm 15 extends in line with one of the ports and coupling arm 15 is moulded onto second port 12.

Measuring means 14 are formed by a pressure sensor arranged on the underside of coupling arm 15. Pressure sensor 14 is provided with coupling pins 14A for insertion in coupling elements 24 on PCB 22. Pressure sensor 14 can be arranged, for instance by glueing, such that it is in contact with the liquid or gas flowing through second port 12 during the measurement. Suitable pressure sensors are known in the relevant field.

In figure 3C pressure sensor 14 is accommodated in a protective cap 15B which can optionally be arranged on coupling arm 15.

Coupling tap 10 is provided adjacently of pressure sensor 14 and close to the outer end of coupling arm 15 with a lock 15A which is intended for co-action with the locking means in opening 21 of connector module 20.

Communication module 100 and/or connector module 20 are further provided with electronic components for reading the measurement values of the pressure sensors and converting thereof to data suitable for transmission. Communication module 100 and/or connector module 20 comprises for this purpose means for digitizing the data. Communication module 100 and/or connector module 20 can also comprise a pre-amplifier and/or a memory. Such electronic components are commercially available and therefore known to a skilled person in the field.

Communication module 100 and/or connector module 20 is also provided with means for attaching the communication module to a patient, such as adhesive electrodes (not shown), and is suitable for stationary and ambulatory use. Communication module 100 and/or connector module 20 is suitable and intended for reuse. Each of the two can be easily cleaned in usual manner, for instance with a wet cloth.

Although the preferred embodiments are illustrated on the basis of a connector module 20 with four openings 21 for connection of four coupling taps 10, it will be apparent that the number of openings 21 on connector module 20 can differ herefrom. A minimum of one coupling opening 21 for coupling means 10, preferably a coupling tap 10, is present on connector module 20. It is further noted that the communication module need not necessarily be provided with a display 101. Communication module 100 can in addition take a different form, for instance be of a manageable size, or a handheld embodiment. Additional electrical connectors, for instance an EMG connector, can optionally be further connectable to PCB 22.

The system according to the invention is described in the context of urological applications. It will however be apparent to a skilled person in the field that the system according to the invention is also suitable for other applications. Several non-limitative examples of other applications are: hemodynamic measurements, (invasive) blood pressure measurements and so on. Gastro-enterological measurements can also be performed using the system according to the invention. As alternative to the flow reduction valve, a thin capillary can be connected to the non-return valve for perfusion.

The invention is of course not limited to the shown and described embodiments but comprises any embodiment falling within the scope of the appended claims as seen in the light of the accompanying drawings.

## Claims

1. System for performing in vivo pressure measurements, comprising one or more pressure sensors, a connector module provided with one or more electrical coupling elements for the one or more pressure sensors and one or more coupling means for a catheter, wherein the one or more coupling means for a catheter are each provided with at least three ports and a tap for coupling two of the three ports as desired, **characterized in that** each of the one or more coupling means is embodied as a coupling module which is releasably connectable to the connector module, and that the pressure sensors are arranged on the coupling modules.

2. System as claimed in claim 1, wherein each coupling module is provided with a coupling arm which extends in line with one of the ports and is configured for locking to the connector module, and wherein the pressure sensor is arranged on the coupling arm and configured for connection to one of the electrical coupling elements on the connector module.

3. System as claimed in claim 2, wherein the pressure sensor is provided with coupling pins for insertion in the electrical coupling element.

4. System as claimed in claim 2 or 3, wherein the connector module is provided with one or more openings for receiving the coupling arm, and wherein the electrical coupling elements are arranged in the openings.

5. System as claimed in one or more of the foregoing claims, wherein each coupling module comprises a snap-lock for releasable connection to the connector module.

6. System as claimed in one or more of the foregoing claims, wherein the coupling module is embodied as two-way tap.

7. System as claimed in one or more of the foregoing claims, wherein one of the ports of one or more of the coupling modules is provided with a non-return valve.

8. System as claimed in one or more of the foregoing claims, wherein the system further comprises a communication module for processing and/or transmitting measurement data from the one or more pressure sensors, which communication module is releasably connectable to the connector module.

9. System as claimed in claim 8, wherein the communication module is provided with a display.

10. Coupling module for a catheter, which coupling module is embodied as two-way tap and is provided with at least three ports and a tap for coupling two of the three ports as desired, wherein the coupling module is provided with a coupling arm extending in line with one of the ports, and wherein a pressure sensor is arranged in the coupling arm.

11. Coupling module for a catheter as claimed in claim 10, wherein the coupling arm is configured for releasable locking to a connector module so as to form a system for performing in vivo pressure measurements, wherein the connector module is provided with at least one electrical coupling element for the pressure sensor.
